# EUROPEAN PATENT SPECIFICATION

(11) **EP 2 538 931 B1**
(45) Date of publication and mention of the grant of the patent: **03.08.2016**
(21) Application number: 11705961.8
(22) Date of filing: 25.02.2011
(51) Int. Cl.: A61K 31/00, A61K 31/635

(54) **PRODUCTS AND METHODS**
PRODUKTE UND VERFAHREN
PRODUITS ET PROCÉDÉS ASSOCIÉS

(30) Priority: 26.02.2010 US 308539 P
(43) Date of publication of application: 02.01.2013
(73) Proprietor: University Of Durham, Old Elvert Durham DH1 3HP (GB)
(72) Inventor: WEINKOVE, David, Durham DH1 3LE (GB)
(74) Representative: Chapman, Paul Gilmour
(86) International application number: PCT/GB2011/000264
(87) International publication number: WO 2011/104515

(56) References cited:
- WO-A2-2006/066244
- WO-A2-2010/011111
- HACKMANN C: "[Observations on influenceability of age phenomena in experimental animals by peroral administration of combinations of 2-(p-aminobenzolsulfonamide)-pyrimidin].", MÜNCHENER MEDIZINISCHE WOCHENSCHRIFT (1950) 21 NOV 1958 LNKD- PUBMED:13622503, vol. 100, no. 47, 21 November 1958 (1958-11-21), pages 1814-1817, XP009147133, ISSN: 0027-2973
- MENG ET AL: "Effects of epigallocatechin-3-gallate on mitochondrial integrity and antioxidative enzyme activity in the aging process of human fibroblast", FREE RADICAL BIOLOGY AND MEDICINE, ELSEVIER SCIENCE, US, vol. 44, no. 6, 15 December 2007 (2007-12-15), pages 1032-1041, XP022510684, ISSN: 0891-5849, DOI: DOI:10.1016/J.FREERADBIOMED.2007.11.023

## Description

Ageing is a multifactorial process characterised by the progressive decrease of the functional capacity of all the tissues and organs of the body and by the declining ability to respond to stress, increasing homeostatic imbalance and increased risk of disease. At present, the biological basis of ageing is not completely understood. Substantial variability exists in the rates of ageing across different species, and that this to a large extent is genetically based. In model organisms and laboratory settings, researchers have been able to demonstrate that selected alterations in specific genes can extend lifespan. Nevertheless, even in the relatively simple organisms, the mechanism of ageing remains to be elucidated.

In addition to genetic ties to lifespan, diet has been shown to substantially affect lifespan in many animals. Specifically dietary (often referred to as caloric) restriction (that is, restricting diet to 30-50% less than an *ad libitum* animal would consume, while still maintaining proper nutrient intake), has been shown to increase lifespan in mice up to 50%. There is a multibillion-dollar industry that produces dietary supplements with the goal of improving human health. However, while a number of specific dietary constituents or manipulations have been postulated to increase human lifespan none have yet been proven.

Defining the precise relationship between diet and ageing is challenging experimentally because of the complexity of diet, confounding environmental factors, and the heterogeneous gastrointestinal microbial flora found in all animals. The metabolism of gastrointestinal microbes has a significant effect on the nutrition of the host animal. There is evidence that variations in microbe composition can have large effects on the animal including enhancing obesity. Microbes assist animal nutrition by converting ingested food to more accessible metabolites and by synthesising essential compounds that the host animal cannot synthesise. However, these inputs from microbial metabolism can make it difficult to interpret effects of diet on lifespan.

To increase understanding of relationships between intestinal microbes, the animal and their diet, the inventors have turned to the nematode *Caenorhabditis elegans* / bacteria *E. coli* model, in which they can control the environment and genetics of both microbe and animal.

Using this approach the inventors determined that a mutant *E. coli* deficient in folate biosynthesis causes a substantial increase in *C. elegans* lifespan by decreasing folate supply to the animal. While not wishing to be constrained to any particular theory, the inventors consider that decreased folate intake as a result of a poor diet or compromised intestinal microbial metabolism may trigger a nutrient-sensing mechanism that increases animal lifespan in conditions unfavourable for growth and reproduction.

Folate (or folic acid; vitamin B₉) is essential to numerous functions in the cell: ranging from nucleotide biosynthesis to the providing methyl donors. The human body needs folate to synthesise DNA, repair DNA, and methylate DNA as well as to act as a cofactor in biological enzymatic reactions involving folate. It is especially important during periods of rapid cell division and growth. Both children and adults require folic acid to produce healthy red blood cells and prevent anemia. Animals cannot synthesise folate, hence they need to obtain it from their diet or intestinal microbes.

Folate is a very important dietary component during periods of rapid cell division and growth, such as infancy and pregnancy. Adequate folic acid intake during the periconceptional period, the time just before and just after a woman becomes pregnant, helps protect against a number of congenital malformations, including neural tube defects, e.g spina bifida and anencephaly.

Since the discovery of the link between insufficient folic acid and neural tube defects (NTDs), governments and health organizations worldwide have made recommendations concerning folic acid supplementation for women intending to become pregnant. This has led to the introduction in many countries of fortification, where folic acid is added to flour with the intention of everyone benefiting from the associated rise in blood folate levels.

Hackmann et al (Observations on influencability of age phenomena in experimental animals by peroral administration of combinations of 2-(p-aminobenzosulfonamide)-pyrimidin; Munchener Medizinische Wochenschrift (1950) 100, 47, 1814-1817) describes that the long term oral administration of compounds of the 2-(p-aminobenzolesulphanamido)-pyrimidin in animals exerts an influence on length of life, body weight and fur quality.

WO 2010/011111 describes a composition for controlling ageing and/or extending lifespan. More specifically, WO 2010/011111 describes a composition including dapsone (which acts as an antioxidant) as an active ingredient for controlling ageing.

WO 2006/066244 describes a method and composition for extending the lifespan of an individual and delaying the onset of age-related disease. In particular this document describes the administration of a dose of oxaloacetate to mimic conditions of calorie restriction.

However, until the present invention it had not been disclosed or suggested that folate levels, particularly reducing folate levels, can have a beneficial effect on animal lifespan. Indeed it can be appreciated from the comments above and other information in the art that, until the present invention, the perception was that dietary folate levels should be elevated to ensure an adequate amount of folate was taken up by the body.

According to an aspect of the invention there is provided an *in vitro* method of screening for an agent for use as a medicament for slowing ageing comprising determining whether a test agent inhibits microbial folate biosynthesis.

Also provided is a non-therapeutic use of an inhibitor to reduce the activity of an enzyme in the folate biosynthesis pathway for slowing ageing, wherein (i) the enzyme is 3-dehydroquinate dehydratase; or (ii) the enzyme is dihydropteroate synthase (DHPS) and the inhibitor is p-aminosalicylic acid.

Also provided is a non-therapeutic method of slowing ageing, the method comprising administering an inhibitor of an enzyme in the folate biosynthesis pathway, wherein: (i) the enzyme is 3-dehydroquinate dehydratase; or (ii) the enzyme is dihydropteroate synthase (DHPS) and the inhibitor is p-aminosalicylic acid.

Described herein is an inhibitor of microbial folate biosynthesis for use as a medicament for slowing ageing.

Also described is an agent capable of reducing folate uptake by an animal for use as a medicament for slowing ageing.

It is to be understood that the inhibitor and agent have a single inventive character in common, in that the inhibitor or agent can be used as a medicament to reduce folate levels in a subject leading to the slowing of ageing. This reduction is effected either by reducing microbial folate biosynthesis, or reducing folate uptake.

The nematode worm, *Caenorhabditis elegans,* has been utilised as a model organism in biological research for the last 40 years. Large scale genetic screens and subsequent molecular analysis are made possible by the fast generation time, small size and ease of culture, including the ability to freeze animals. The genome is fully sequenced and available mutants disrupt a large number of genes. With a lifespan of only a few weeks, *C. elegans* is an established model for ageing research. Lifespan can be extended considerably by a number of mechanisms. For example, mutations in a pathway homologous to the mammalian insulin/insulin-like growth factor signalling pathway result in a large increase in lifespan. This work led to experiments that showed that mice bearing mutants in this pathway also have an extended lifespan. A well-studied intervention shown to increase lifespan in several animal models is dietary restriction, also known as calorie restriction.

Dietary intake is limited to that needed to avoid malnutrition. Restricting the food intake of C. *elegans* through mutants that eat more slowly or by reducing food availability also results in increased lifespan. Nevertheless, the mechanism of dietary restriction is still not well understood and it is still not clear what constituents of the diet must be restricted.

A key advantage of *C*. *elegans* as an animal model for diet/lifespan studies is that all microbes can be removed from the intestine and microbes can be limited to a single species. *C. elegans* is normally fed a lab strain of *Escherichia coli*, derived from a strain originally found in the human intestine. While not the natural food of *C*. *elegans*, *E. coli* provides good nutrition as measured by the rate of development. This rate of development cannot be achieved with media lacking live microbes, including killed *E*. *coli.* Thus it is likely that active microbial metabolism is vital for full nutrition.

In the present invention, the inventors were interested in understanding further the relationship between *C*. *elegans* diet and lifespan. They identified a strain of *E. coli* which, when used as sole foodsource for *C*. *elegans*, lead to the animal having a 30 to 50% increase in lifespan.

The inventors therefore consider that inhibitors of microbial folate biosynthesis can be used as medicaments for slowing ageing.

By "slowing ageing" as used herein we include that the inhibitors and other agents of the invention act to slow down the biological processes which cause an organism to age. As discussed above, ageing is characterised by the progressive decrease of the functional capacity on all the tissues and organs of the body; ageing is characterized by the declining ability to respond to stress, increasing homeostatic imbalance and increased risk of disease.

Included within the term "slowing ageing" is that the inhibitors and other agents of the invention act to prevent age related diseases. The term "age-related disease" is used herein to refer to diseases, conditions and symptoms that are predominantly found or manifested in older animals, e.g., in humans, people over 50 or more preferably people over 65. For any animal, age-related diseases would manifest after maturation, e.g., post-development. The age at which maturity is reached is different depending on the animal and for each animal such time would be well known to those of skill in the art. Age-related diseases include certain cancers, cardiovascular disease, atherosclerosis, hypertension, diabetes (e.g., type 2), osteoporosis, depression, neurodegenerative disease, Alzheimer's, Parkinson's, glaucoma, certain immune system defects, kidney failure, liver steatosis, and other conditions well known to those of skill in the art.

Moreover, the inhibitors and other agents of the invention also act to increase lifespan, which can be considered to be another effect of slowing ageing. The term "increasing lifespan" as used herein, refers to the length of a subject's life, e.g., the number of years, months, weeks, days, minutes, etc., in the lifespan of an animal.

As used herein, by "slowing ageing" and an "increase" of lifespan longevity, this can mean a delay in the onset of age-related diseases and/or conditions and/or a delay and/or stabilization of the aging process. It is noted that the inventors have determined that *C*. *elegans* maintained on a diet of reduced folate show a sustainable increase in lifespan of between 30% to 50%. While it could be the case that humans or domesticated animals when treated with medicaments of the invention do not show such difference, it is anticipated that organisms treated with inhibitors and other agents of the invention will live for several years longer; for humans, this could be more than 10 years longer.

The subjects of the invention are typically humans or domesticated animals when treated with medicaments of the invention, and non-human animals, e.g., rodents, nematodes, or flies, when screening methods are employed. Exemplary non-human animals include, but are not limited to, insects (e.g., *Drosophila,* including *Drosophila melanogaster*), nematodes (e.g., *Caenorhabditis elegans*), mammals, non-human primates, rodents (e.g., mice, rats, and hamsters), stock and domesticated animals (e.g., pigs, cows, sheep, horses, cats, and dogs), and birds. In certain embodiments, animals amenable to transformation techniques are particularly useful.

As mentioned above, an important source of folate for animals is from intestinal microbes. Bacterial folate synthesis has been well studied. A schematic diagram is provided in the accompanying figures.

Further investigations by the inventors determined that the specific strain of *E*. *coli* had a mutation in the *aroD* gene which would lead the synthesis of a truncated enzyme. The *C*. *elegans* lifespan effect was rescued by transformation of the mutant bacteria with the plasmid containing *aroD.* Thus, mutation of the *E. coli aroD* gene causes a slowing of *C*. *elegans* ageing.

*aroD* encodes the enzyme 3-dehydroquinate dehydratase, a core component of the shikimic acid pathway. The end product of this pathway is chorismate, which acts as a precursor to all aromatic compounds in the bacterial cell, including the aromatic amino acids, the folate precursor para-amino benzoic acid (PABA) and quinones. The mutation in the gene *aroD* disrupts the shikimic acid pathway, which generates aromatic compounds. Despite the large number of compounds that depend on the shikimic acid pathway for their synthesis, addition of only one, the folate precursor para-aminobenzoic acid (PABA), eliminates the increased lifespan effect. Moreover, *C*. *elegans* lifespan can be increased by inhibition of the bacterial folate biosynthesis pathway using a sulphonamide compound, which acts as a competitive inhibitor with PABA in the folate biosynthesis pathway.

Hence the inventors have identified that reduced bacterial synthesis of folate slows ageing.

Folates are tripartite molecules comprising pterin, para-aminobenzoic acid, and glutamate. The folate biosynthesis pathway generates dihydrofolate (DHF) and tetrahydrofolate (THF). There are many species of folate molecules, but generally folate molecules have to be reduced to THF to be biologically active. The pathway consists of two branches that converge to form folate: one where para-amino benzoic acid (PABA) is formed from chorismate, and another where hydroxymethyl dihydropterin pyrophosphate (HMDPP) is formed from the nucleotide Guanosine-5'-triphosphate (GTP). PABA and HMDPP are then fused to become dihydropteroate. Dihydropteroate is in turn converted by an addition of a glutamate moiety to DHF. Since DHF is not an active form of folate, it has in turn to be reduced to THF by dihydrofolate reductase.

As referred to herein, by "microbial folate biosynthesis" we include the above mentioned pathway leading to the production of folate. Specific parts of this pathway will now be discussed. Where relevant, reference to an "EC" number relates to an enzyme nomenclature given to that specific enzyme according to the Nomenclature Committee of the International Union of Biochemistry and Molecular Biology (NC-IUBMB; http://www.chem.qmul.ac.uk/iubmb/ or http://www.brenda-enzymes.org/). Specific details of such designated enzymes can be readily identified from IUMBM information. For example, *aroD* encodes the enzyme 3-dehydroquinate dehydratase. 3-dehydroquinate dehydratase has a reference EC 4.2.1.10. Entering this reference number in to the http://www.brenda-enzymes.org provides access to a very large amount of information concerning this enzyme, including: nucleotide and polypeptide sequences; inhibitory compounds; organisms in which it is found; substrate(s) and products(s). Hence from the information provided herein, data concerning at least the sequence; species; enzymatic reactions; products; substrates; arid inhibitors can be rapidly identified.

One part of the folate biosynthesis pathway requires the production of para-amino benzoic acid (PABA), as described below.

Phosphoenolpyruvate and erythrose-4-phosphate react to form 3-deoxy-D-arabinoheptulosonate-7-phosphate (DAHP), in a reaction catalysed by the enzyme DAHP synthase (EC 2.5.1.54). DAHP is then transformed to 3-dehydroquinate (DHQ), in a reaction catalysed by DHQ synthase (EC 4.2.3.4). DHQ is dehydrated to 3-dehydroshikimate by the enzyme dehydroquinase dehydratase (*aroD*; (EC 4.2.1.10)), which is reduced by to shikimic acid by the enzyme shikimate dehydrogenase (EC 1.1.1.25). Shikimate is then converted to shikimate-3-phosphate by shikimate kinase (EC 2.7.1.71), and then on to 5'-enolpyruvyl-shikimate-3-phosphate by EPSP synthase (EC 2.5.1.19). 5'-enolpyruvyl-shikimate-3-phosphate is then transformed to chorismate by the action of chorismate synthase (EC 4.2.3.5). Finally, chorismate is aminated to aminodeoxychorismate (ADC) by ADC synthease (EC 2.6.1.85), then ADC is converted on to PABA by ADC lyase (EC 4.1.3.38).

The second branch of the pathway is where hydroxymethyl dihydropterin pyrophosphate (HMDPP) is formed from GTP, as described below.

GTP cyclohydrolase (EC 3.5.4.16) catalyzes a complex reaction in which the five-membered imidazole ring of GTP is opened, C8 is expelled as formate, and a six-membered dihydropyrazine ring is formed as 7,8-dihydroneopterin triphosphate. 7,8-dihydroneopterin triphosphate is then converted to the corresponding monophosphate by a pyrophosphatases. Dihydroneopterin aldolase (DHNA; EC 4.1.2.25) then converts dihydroneopterin to hydroxymethyl dihydroneopterin, which in turn is pyrophosphorylated by hydroxymethyl dihydropterin pyrophosphokinase (HPPK; EC .2.7.6.3) to form hydroxymethyl dihydropterin pyrophosphate (HMDPP).

Following this, PABA is condensed with HMDPP by the action of dihydropteroate synthase (EC 2.5.1.15), a key enzyme in the microbial folate biosynthesis pathway as discussed below, to form dihydropteroate. Dihydropteroate is glutamylated by dihydrofolate synthase (EC 6.3.2.12) giving dihydrofolate. Dihydrofolate is finally converted to tetrahydrofolate (THF) by the action of dihydrofolate reductase (EC 1.5.1.3), another key enzyme in the microbial folate biosynthesis pathway as discussed below. Folylpolyglutamate synthase (FPGS; EC 6.3.2.17) then can add a γ-glutamyl tail to THF.

The inventors have determined that a disruption in the formation of PABA, and separately the inhibition of dihydropteroate synthase (which condenses PABA with HMDPP to form dihydropteroate), both can lead to a slowing in ageing. Thus it can be seen that inhibition of different parts of the microbial folate biosynthesis pathway can lead a slowing in ageing.

Consequently, the inhibitors described herein can target one or more parts of the microbial folate biosynthesis pathway as discussed above to act to slow ageing.

By "inhibitor of microbial folate biosynthesis", a preferred embodiment of this aspect of the invention is wherein the agent inhibits the activity of an enzyme in the folate biosynthesis pathway; or, possibly, more than one enzymes in this pathway.

From the information above, it can be readily understood which enzymes are considered to be part of the folate biosynthesis pathway. As would also be understood, the enzymes and associated nomenclature given relate to proteins which catalyse specific reactions. There may be variations between the amino acid sequences of enzymes from different species, as would be expected; however this embodiment of the invention generally relates to inhibiting any example of such enzymes in a suitable microbial species.

The enzyme may be 3-dehydroquinate dehydratase (EC 4.2.1.10). As stated above, 3-dehydroquinate dehydratase is encoded by the E. coli gene aroD. The inventors have determined that E. coli having a disruption in aroD leads to *E. coli* which when used as a food source for *C*. *elegans* increases the lifespan of those animals. Accordingly therefore, in this embodiment of the invention it is preferred that the inhibitor reduces the activity of this enzyme.

As an alternative the enzyme is dihydropteroate synthase (DHPS). DHPS catalyses the condensation of PABA and hydroxymethyl pterin pyrophosphate to dihydropteroate. It is therefore the enzyme which combines the two key branches of folate synthesis. It is one of the two most well studied enzymes in this pathway since it is the target for antibiotics.

DHPS inhibitors have been the focus of much development in the past, since this is an enzyme only present in bacteria and is essential for their maintenance. Sulphonamides are a key class of DHPS inhibitors. They were among the first antibiotics developed. They inhibit DHPS by mimicking the substrate PABA, thereby competing with that substrate for DHPS. Examples of sulphonamides include sulphanilamide (CAS No. 63-74-1) ; sulfamethoxazole (CAS No. 723-46-6) ; sulfamethazine (CAS No. 57-68-1) ; sulfadimethoxine (CAS No. 122-11-2); sulfamethoxypyridazine (CAS No. 80-35-3) ; sulfacetamide (CAS No. 144-80-9); sulfadoxin (CAS No. 2447-57-6); sulfamerazine (CAS No. 127-79-7); sulfadiazine (CAS No. 68-35-9); sulfapyridine (CAS no. 144-83-2); sulfathiazole (CAS No. 72-14-0). All available from Sigma-Aldrich.

Sulfonamides were the first antimicrobial drugs, and paved the way for the antibiotic revolution in medicine. They were the first and only effective antibiotics in the years before penicillin. Sulfonamides are poorly absorbed by the body, and this is one of the reasons for their decline in use since the development of penicillin and other modern antibiotics. However, this is an advantage for the present invention. This is because the inhibitor of the first aspect of the invention is to microbial folate biosynthesis, and hence it is desirable that the inhibitor targets microbes of the gastointestinal tract flora and is not absorbed by the human or animal body. Moreover, sulphonamides are not very effective antimicrobial agents since they are unlikely to kill the microbe. However, again for the present invention this is an advantage since it is preferably only to inhibit folate biosynthesis, not to stop it completely.

If the inhibitor is a sulphonamide it may preferably be sulfamethoxazole.

An alternative DHPS inhibitor is the PABA analogue p-aminosalicylic acid (PAS, CAS No. 65-49-6).

As used herein, by "inhibitor" we mean an agent that causes a reduction in the amount or rate of production of microbial folate.

Folate is an essential molecule for microbial cells. Hence it is to be understood that the inhibitor of this aspect of the invention preferably does not completely inhibit folate biosynthesis, otherwise the microbial cells are likely to die. Accordingly, by 'inhibit' we mean that the inhibitor reduces microbial folate synthesis by approximately 10%, 15%, 20%, 25%, 30%, 35%, 40%, 45%, 50%, 55%, 60%, 65%, 70%, 75%, 80%, 85%, 90% or 95%. Preferably the inhibition is in the range of 10% to 90%, for example 25% to 75% reduction, or around 50% reduction, in folate biosynthesis.

An "inhibitor" of microbial folate biosynthesis can act via a number of different mechanisms.

For example, the inhibitor could be a "competitive inhibitor" for an enzyme in the pathway, much as the sulphonamides act as DHPS inhibitors. Such inhibitors are typically small molecules which mimic the structure of a substrate of the enzyme. Alternatively the inhibitors may irreversibly modify an enzyme, often by permanently altering the active site of the enzyme in the pathway.

Another form of "inhibitor" is that which reduces the expression and/or activity of a given gene, protein, polypeptide, mRNA, or the like, e.g., to produce a reduction in microbial folate biosynthesis. Hence the term "inhibitor" can also refer to a reduction in an expression level, activity or property of the gene, protein, etc. For example, reduction can cause a decrease in a protein activity (e.g., catalytic activity) or binding characteristic. Inhibition can, e.g., cause a decrease in expression of one or more genes, e.g., a change in transcription level, a change in stability of an mRNA that encodes a polypeptide, a change in translation efficiency, and/or a change in protein stability. Change in apparent protein activity can arise from changes in its expression; as additional examples, an inhibitor can change (activate or inhibit) activity of the polypeptide itself, for example, by binding to the polypeptide and stabilizing a catalytically active conformation thereof, by binding to and inhibiting the polypeptide, or by affecting post-translational modification of the polypeptide and thus changing its activity.

Examples of such inhibitors can therefore include ligands, small molecules, antibodies, agonists, antagonists, complexes thereof, interfering RNAs (double-stranded RNAs as well), of the components of microbial folate biosynthesis.

As mentioned above, animals cannot synthesise folate, hence they need to obtain it from their diet or intestinal microbes.

As referred to herein, by "microbial" we include folate biosynthesis in any microorganism. In the above section describing the folate biosynthetic pathway, information is provided to allow the skilled person to identify the components of that pathway from a wide range of different microbes.

However, preferably by "microbe" we include where the microbe is part of the gastointestinal tract flora.

In humans (and likely many mammals) between 300 and 1000 different species live in the gut with most estimates at about 500. However, it is probable that 99% of the bacteria come from about 30 or 40 species. Fungi and protozoa also make up a part of the gut flora, but little is known about their activities. Not all the species in the gut have been identified, and populations of species vary widely among different individuals but stay fairly constant within an individual over time, even though some alterations may occur with changes in lifestyle, diet and age.

However, it is known that most bacteria belong to the genera *Bacteroides, Clostridium, Fusobacterium Eubacterium, Ruminococcus, Peptococcus, Peptostreptococcus,* and *Bifidobacterim.* Other genera such as *Escherichia* and *Lactobacillus* are present to a lesser extent. Species from the genus *Bacteroides* alone constitute about 30% of all bacteria in the gut, suggesting that this genus is especially important in the functioning of the host. The currently known genera of fungi of the gut flora include *Candida*, *Saccharomyces, Aspergillus,* and *Penicillium.*

To minimise side effects preferably the inhibitor of microbial folate biosynthesis would be used at a minimum concentration that would significantly reduce the level of microbial folates, which could be monitored by measuring folate levels in the faeces.

The point of action of the inhibitor is to increase lifespan is the intestinal microbe, and not the host. Therefore it would be desirable to modify the inhibitor (for example, SMX or other sulfonamides) so that they are not absorbed by the host but retain the ability to inhibit bacterial folate synthesis.

Hence the inhibitor of microbial folate biosynthesis may not be absorbed by the subjects of the invention; preferably the inhibitor of microbial folate biosynthesis is not absorbed by mammals. By "not absorbed" we mean that at least 80%, 85%, 90%, 95%, 96%, 97%, 98%, 99% or more of the inhibitor is not absorbed by the subjects of the invention.

An example of such a type of modification is the drug Rifaximin, which is a non-absorbable version of the antibiotic rifamycin. Rifaximin was prepared by the addition of a pyridoimidazole ring to rifamycin. This modification allows an increase in the gut concentration of this drug while avoiding side effects of systemic uptake.

Similar approaches may be taken to derive derivatives of the inhibitor of microbial folate biosynthesis so that they are not absorbed by the host but retain the ability to inhibit bacterial folate synthesis.

In addition, since the point of action of the inhibitor is the intestinal microbe, it would be desirable to modify the inhibitor (for example, SMX or other sulfonamides) so that they are not released to the environment until the drug has reached the required location in the gastrointestinal tract.

Hence the inhibitor of microbial folate biosynthesis may be formulated for release in the gastrointestinal tract.

Methods of preparing controlled release formulations of medicaments, (such as SMX or other sulfonamides) are well known in the art and can be readily adapted by the skilled person to prepare inhibitors formulated for a release in the gastrointestinal tract.

There is also provided an agent capable of reducing folate uptake by an animal for use as a medicament for slowing ageing.

The present inventors investigated whether supplementing *C. elegans* maintained on the *aroD E.coli* mutant with folate would suppress lifespan extension. Folic acid supplementation reduced the *C. elegans* lifespan extension substantially but not completely, suggesting that folate is required in both the animal and the microbe for normal lifespan and that decreased folate supply to the animal slows ageing.

There are two routes by which animals can obtain folate: from microbial folate biosynthesis, and from the diet. However, regardless of the source, the animal body still needs to absorb folate, most likely from the gastointestinal tract.

Hence preferably the agent of this aspect of the invention reduces folate uptake by the animal from the gastointestinal tract. By "uptake" we mean the amount of folate absorbed by the body; for example, taken in to the cells in the body.

It is to be understood that the inhibitor and agent have a single inventive character in common, in that the inhibitor or agent can be used as a medicament to reduce folate levels in a subject leading to the slowing of ageing. This reduction is effected either by reducing microbial folate biosynthesis, or reducing folate uptake.

As mentioned above, folate is an essential molecule for animals. Hence it is to be understood that the agent preferably does not completely reduce folate uptake by the animal, otherwise the animal is likely to suffer from related disorders. Accordingly, by 'reduce' we mean that the agent reduces folate uptake by approximately 10%, 15%, 20%, 25%, 30%, 35%, 40%, 45%, 50%, 55%, 60%, 65%, 70%, 75%, 80%, 85%, 90% or 95%. Preferably the reduction is in the range of 10% to 90%, for example 25% to 75% reduction, or around 50% reduction, in folate uptake.

The "agent" can be the same type of inhibitory molecules discussed above in relation to the inhibitors. Preferably the agent is a small molecule; or a biopolymer, preferably a peptide or antibody.

A "small molecule" is a low molecular weight organic compound which is by definition not a polymer. The term small molecule, especially within the field of pharmacology, is usually restricted to a molecule that also binds with high affinity to a biopolymer such as protein, nucleic acid, or polysaccharide and in addition alters the activity or function of the biopolymer. The upper molecular weight limit for a small molecule is approximately 800 Daltons which allows for the possibility to rapidly diffuse across cell membranes so that they can reach intracellular sites of action. In addition, this molecular weight cutoff is a necessary but insufficient condition for oral bioavailability.

Small molecules can have a variety of biological functions, serving as cell signaling molecules, as tools in molecular biology, as drugs in medicine, as pesticides in farming, and in many other roles. These compounds can be natural (such as secondary metabolites) or artificial (such as antiviral drugs); they may have a beneficial effect against a disease (such as drugs) or may be detrimental (such as teratogens and carcinogens).

Biopolymers such as nucleic acids, proteins, and polysaccharides (such as starch or cellulose) are not small molecules, although their constituent monomers - ribo- or deoxyribonucleotides, amino acids, and monosaccharides, respectively - are often considered to be. Very small oligomers are also usually considered small molecules, such as dinucleotides, peptides such as the antioxidant glutathione, and disaccharides such as sucrose.

Folate uptake is mediated by the activity of a folate transporter. The *C. elegans* folate transporter *folt-1* was identified several years ago. Inhibition of the activity of *folt-1* causes a reduction in folate uptake by *C*. *elegans*. *Folt-1* has GenBank reference number EF409995.1, which entry provides details of the amino acid sequence of the protein; nucleotide sequence encoding that protein; and homologues of that protein found in other animals, including humans and domesticated animals.

Preferably the agent inhibits the activity of a folate transporter molecule which is active in the gastointestinal tract; i.e. the transporter has a role in folate uptake by the animal from the gastointestinal tract. For example, a human proton-coupled, high-affinity folate transporter that recapitulates properties of folate transport and absorption in intestine and in various cell types at low pH has been identified: PCFT/HCP1 (GenBank accession Q96NT5). Agents can be developed to target the activity of this particular transporter.

Inhibitors of folate uptake are known. They include sulfasalazine (CAS No, 599-79-1); folinic acid (CAS No. 1492-18-8); 4,4'-diisothiocyanatostilbene-2,2'-disulfonic acid (DIDS, CAS No. 207233-90-7); 4-acetamido-4'-isothiocyanostilbene-2,2'-disulfonic acid (SITS, CAS No. 51023-76-8); or methotrexate (MTX, CAS No. 59-05-2). Such compounds can be used as the agents mentioned above.

Further inhibitors can be readily determined by the person skilled in the art. For example, from the information provided herein and in the GenBank accession, specific antibody molecules can be devised which bind to and prevent the function of a folate transporter in a species of interest.

In an alternative the agent binds to folate and prevents its uptake by the animal.

It can be readily understood by the skilled person that various molecules can be used as folate "scavengers", i.e. they bind to folate and reduce its uptake by the animal from the gastointestinal tract. Such molecules may, for example, be derived from the part of a folate transporter molecule which binds to folate; or from one of a number of different enzymes, e.g. those mentioned above, which bind to folate.

The present invention relates to a range of different molecules which can be used as inhibitors and agents for the purposes stated above. Especially where such molecules are chemicals rather than biological molecules, it is to be understood that the disclosure also relates to derivatives, precursors, and pro-drug versions of such agents.

A further aspect of the invention provides a composition comprising an inhibitor of microbial folate biosynthesis and one or more further agents capable of slowing ageing, wherein said one or more further agents capable of slowing ageing is resveratrol; acetyl-L-carnitine; alpha-lipoic acid; MK-677; and/or rapamycin.

It has been suggested that other agents may be of use in slowing ageing. For example, resveratrol, a chemical found in red grapes, has been shown to extend the lifespan of yeast by up to 60%, worms and flies by up to 30% and one species of fish by almost 60%. Feeding aged rats a combination of acetyl-L-carnitine and alpha-lipoic acid produced a rejuvenating effect. The drug MK-677 has been shown to restore 20% of muscle mass lost due to ageing in humans aged 60 to 81. In addition, rapamycin, has been found to extend the life expectancy of 20-month-old mice by up to 38%. Further compounds that are agonists of sirtuins, other caloric restriction mimetics and other TOR pathway inhibitors are being developed in an attempt to slow the onset of ageing-related disease.

Hence such compounds can be combined with the inhibitor to provide a composition which can be used to slow ageing.

It will be appreciated that the amount of an agent or inhibitor needed according to the invention is determined by biological activity and bioavailability which in turn depends on the mode of administration and the physicochemical properties of the agent or inhibitor. The frequency of administration will also be influenced by the abovementioned factors and particularly the half-life of the agent or inhibitor within the target tissue or subject being treated.

Known procedures, such as those conventionally employed by the pharmaceutical industry (e.g. *in vivo* experimentation, clinical trials etc), may be used to establish specific formulations of the agent or inhibitor and precise therapeutic regimes (such as daily doses and the frequency of administration).

Generally, a daily dose of between 0.01 g/kg of body weight and 0.1g/kg of body weight of an agent or inhibitor may be used in a treatment regimen; more preferably the daily dose is between 0.01mg/kg of body weight and 100mg/kg of body weight.

By way of example a suitable dose of an antibody is 10g/kg of body weight; 1g/kg of body weight; 100mg/kg of body weight, more preferably about 10mg/kg of body weight; and most preferably about 6mg/kg of body weight.

Daily doses may be given as a single administration (e.g. a single daily injection or a single dose from an inhaler). Alternatively the agent or inhibitor (e.g. an antibody or aptamer) may require administration twice or more times during a day.

Medicaments should comprise a therapeutically effective amount of the agent or inhibitor and a pharmaceutically acceptable vehicle.

A "therapeutically effective amount" is any amount of an agent or inhibitor which, when administered to a subject leads to a slowing of ageing.

A "pharmaceutically acceptable vehicle" as referred to herein is any physiological vehicle known to those skilled in the art as useful in formulating pharmaceutical compositions.

In one embodiment, the medicament may comprise between about 0.01 µg and 0.5 g of the agent or inhibitor. More preferably, the amount of the agent or inhibitor in the composition is between 0.01 mg and 200 mg, and more preferably, between approximately 0.1 mg and 100 mg, and even more preferably, between about 1mg and 10mg. Most preferably, the composition comprises between approximately 2mg and 5mg of the agent or inhibitor.

Preferably, the medicament comprises approximately, 0.001%, 0.01%, 0.1% (w/w) to 90% (w/w) of the agent, and more preferably, 1% (w/w) to 10% (w/w). The rest of the composition may comprise the vehicle.

Nucleic acid agents can be delivered to a subject by incorporation within liposomes, alternatively the "naked" DNA molecules may be inserted into a subject's cells by a suitable means e.g. direct endocytotic uptake. Nucleic acid molecules may be transferred to the cells of a subject to be treated by transfection, infection, microinjection, cell fusion, protoplast fusion or ballistic bombardment. For example, transfer may be by ballistic transfection with coated gold particles, liposomes containing the DNA molecules, viral vectors (e.g. adenovirus) and means of providing direct DNA uptake (e.g. endocytosis) by application of the DNA molecules directly to the target tissue topically or by injection.

The antibodies, or functional derivatives thereof, may be used in a number of ways. For instance, systemic administration may be required in which case the antibodies or derivatives thereof may be contained within a composition which may, for example, be ingested orally in the form of a tablet, capsule or liquid. It is preferred that the antibodies, or derivatives thereof, are administered by injection into the blood stream. Injections may be intravenous (bolus or infusion) or subcutaneous (bolus or infusion). Alternatively the antibodies may be injected directly to the liver.

Nucleic acid or polypeptide therapeutic entities may be combined in pharmaceutical compositions having a number of different forms depending, in particular on the manner in which the composition is to be used. Thus, for example, the composition may be in the form of a powder, tablet, capsule, liquid, ointment, cream, gel, hydrogel, aerosol, spray, micelle, transdermal patch, liposome or any other suitable form that may be administered to a person or animal. It will be appreciated that the vehicle of the composition should be one which is well tolerated by the subject to whom it is given, and preferably enables delivery of the therapeutic to the target cell, tissue, or organ.

In a preferred embodiment, the pharmaceutical vehicle is a liquid and the pharmaceutical composition is in the form of a solution. In another embodiment, the pharmaceutical vehicle is a gel and the composition is in the form of a cream or the like.

Compositions comprising such therapeutic entities may be used in a number of ways. For instance, systemic administration may be required in which case the entities may be contained within a composition that may, for example, be ingested orally in the form of a tablet, capsule or liquid. Alternatively, the composition may be administered by injection into the blood stream. Injections may be intravenous (bolus or infusion) or subcutaneous (bolus or infusion). The entities may be administered by inhalation (e.g. intranasally).

Therapeutic entities may also be incorporated within a slow or delayed release device. Such devices may, for example, be inserted on or under the skin, and the compound may be released over weeks or even months. Such devices may be particularly advantageous when long term treatment with an entity is required and which would normally require frequent administration (e.g. at least daily injection).

As mentioned above, while not wishing to be constrained to any particular theory, the inventors consider that decreased folate intake slows ageing.

In addition to their use as human or domestic animal medicaments, the inhibitors or agents described above can also be supplied in the form of a food supplement, additive, functional food, or nutraceutical which can be taken for the purpose of slowing ageing.

The terms "food supplement, additive, functional food, or nutraceutical" generally refer to a food or food extract that has a medicinal effect on human health. The nutraceutical can be contained in a medicinal format such as a capsule, tablet, or powder in a prescribed dose, comprising the inhibitors or agents described above.

Such a food supplement, additive, functional food, or nutraceutical may be specifically designed and targeted towards a particular age, especially in view of the requirement for folate in infancy and pregnancy. For example, such products could be for "aged" human subjects, e.g. over 30, 40, 50, 60, 70 80 or 90 years old.

The amount of inhibitors or agents formulated into the food supplement, additive, functional food, or nutraceutical will necessarily vary depending on the nature of the inhibitors or agents to be used. As way of guidance, approximately, 0.001%, 0.01%, 0.1% (w/w) to 90% (w/w) of the agent, and more preferably, 1% (w/w) to 10% (w/w).

There is also described a maintenance food characterised by comprising low levels of folate.

"Maintenance food" as used herein particularly refers to those foods used for domesticated pets, such as dogs or cats. Such foods typically at least a maintenance amount of the macronutrients (protein, carbohydrate, lipid and fiber) required, according to guidelines published and from time to time updated by bodies such as NRC (National Research Council) and AAFCO (Association of American Feed Control Officials).

In use, typically as far as possible such foods are used as the sole source of food for the animal e.g., a daily amount, of one or more maintenance foods that together satisfy substantially the full nutritional requirement for maintenance of the animal. The food is not necessarily constant during the period of such feeding so long as substantially the full nutritional requirement for maintenance of the animal is satisfied.

In this way, and in contrast to humans, it is possible to regulate the amount of folate supplied to the animal.

The amount of folate in the maintenance food can be a percentage of the R.D.A. (recommended daily allowance) of folate for the animal. For humans, the R.D.A. is 400µg. The National Research Council (NRC) recommends diets containing 0.2 mg and 1.0 mg of folic acid (I) per kg of dry diet (assuming 5 kcal metabolizable energy per gram) for dogs and cats, respectively.

Hence the amount of folate may be between 10%, 15%, 20%, 25%, 30%, 35%, 40%, 45%, 50%, 55%, 60%, 65%, 70%, 75%, 80%, 85%, 90% or 95% of the R.D.A.

Preferably the amount is in the range of 10% to 90%, for example 25% to 75% reduction, or around 50% of the R.D.A.

The maintenance food may further comprise one or more inhibitors, agents or compositions as described above. The amount of inhibitors or agents formulated into the maintenance food will necessarily vary depending on the nature of the inhibitors or agents to be used. As way of guidance, approximately, 0.001%, 0.01%, 0.1% (w/w) to 90% (w/w) of the agent, and more preferably, 1% (w/w) to 10% (w/w) can be used.

An aspect of the invention provides an *in vitro* method of screening for an agent for use as a medicament for slowing ageing comprising determining whether a test agent inhibits microbial folate biosynthesis.

An embodiment of this aspect of the invention is wherein the method comprises the steps of (i) contacting a population of micro-organisms with a quantity of the test agent; and (ii) determining the effect of the test agent on microbial folate biosynthesis.

A further embodiment of this aspect of the invention is wherein the micro-organism is a member of the animal gastointestinal tract flora.

Also described is a method of screening for an agent for use as a medicament for slowing ageing comprising determining whether a test agent reduces folate uptake by a non-human animal.

The method may comprise the steps of (i) contacting a non-human animal with a quantity of the test agent; and (ii) determining the effect of the test agent on folate uptake by the non-human animal.

The agent may reduce folate uptake by the non-human animal from the gastointestinal tract.

The methods described above are "screening methods" to identify agents for use as a medicament for slowing ageing. For the reasons outlined above, an agent that inhibits microbial folate biosynthesis, or reduces folate uptake by a non-human animal, could be of use in as a medicament for slowing ageing.

In order to assess whether the test agent inhibits microbial folate biosynthesis, or reduces folate uptake by a non-human animal, it is useful to compare the amount of microbial folate biosynthesis or folate uptake, in a micro-organisms or non-human animal exposed to a test compound to a "reference sample", i.e. micro-organisms or non-human animal not exposed to a test compound.

Hence the test agent may produce an increase, decrease or no effect on microbial folate biosynthesis or folate uptake.

The step of "determining the effect of the test agent on microbial folate biosynthesis" may be performed using a number of different experimental techniques.

The amount of folate produced by a population of micro-organisms can be measured using HPLC-MS; a standard laboratory technique well known by the skilled person.

The step of "determining the effect of the test agent on folate uptake by the non-human animal" may be performed using a number of different experimental techniques. For example, a laboratory test animal can be supplied with a source of labelled PABA, in conjunction with one or more test agents. The amount of labelled PABA taken up in to the animal body, compared to a control animal, can then be used as a measure of the effect of the test agent on folate uptake.

The screening methods of the invention relates to screening methods for drugs or lead compounds. The test agent may be a drug-like compound or lead compound for the development of a drug-like compound.

The term "drug-like compound" is well known to those skilled in the art, and may include the meaning of a compound that has characteristics that may make it suitable for use in medicine, for example as the active ingredient in a medicament. Thus, for example, a drug-like compound may be a molecule that may be synthesised by the techniques of organic chemistry, less preferably by techniques of molecular biology or biochemistry, and is preferably a small molecule, which may be of less than 5000 daltons and which may be water-soluble. A drug-like compound may additionally exhibit features of selective interaction with a particular protein or proteins and be bioavailable and/or able to penetrate target cellular membranes, but it will be appreciated that these features are not essential.

The term "lead compound" is similarly well known to those skilled in the art, and may include the meaning that the compound, whilst not itself suitable for use as a drug (for example because it is only weakly potent against its intended target, non-selective in its action, unstable, poorly soluble, difficult to synthesise or has poor bioavailability) may provide a starting-point for the design of other compounds that may have more desirable characteristics.

The screening methods of the invention can be used in "library screening" methods, a term well known to those skilled in the art. Thus, for example, the methods of the invention may be used to detect (and optionally identify) a test agent that inhibits microbial folate biosynthesis, or reduces folate uptake. Aliquots of a library may be tested for the ability to give the required result. Hence by "test compound", we include where assays are performed in which more than one compound at a time is assessed, as is commonly performed in high throughput screening assays well known in the art.

An embodiment of the screening methods of the invention is wherein the method further comprises the step of selecting an agent that inhibits microbial folate biosynthesis, or reduces folate uptake By "inhibits" and "reduces" we include where microbial folate biosynthesis, or folate uptake, is reduced by approximately 10%, 15%, 20%, 25%, 30%, 35%, 40%, 45%, 50%, 55%, 60%, 65%, 70%, 75%, 80%, 85%, 90% or 95%. Preferably the range is 10% to 90%, for example 25% to 75% reduction, or around 50% reduction.

An embodiment of the screening methods of the invention is wherein the method has the additional step of mixing the selected agent (or a derivative or analogue thereof) with a pharmaceutically acceptable vehicle.

A further aspect of the invention provides a method of making a pharmaceutical composition comprising the screening method of the invention and the additional step of mixing the selected agent (or a derivative or analogue thereof) with a pharmaceutically acceptable carrier.

All of the features described herein (including any accompanying claims, abstract and drawings), and/or all of the steps of any method or process so disclosed, may be combined with any of the above aspects in any combination, except combinations where at least some of such features and/or steps are mutually exclusive.

The invention will now be further described with reference to the following examples and Figures.

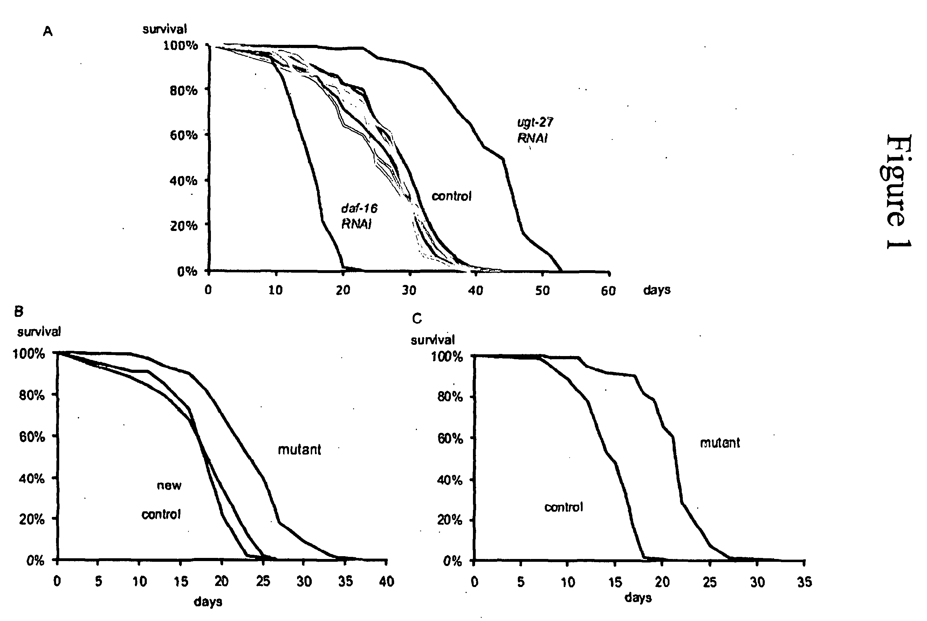

Figure 1: Discovery of the mutant. A). Survival curves of *rrf-3(pk1426); daf-*2(m577) animals on various RNAi strains (25°C). The control strain (black, n=201) is HT115(DE3) containing the empty vector L4440. One strain containing the plasmid for the *ugt-27* gene (n = 92) caused a 50% increase in lifespan (P<.0001). Other plasmids were for other drug metabolizing enzyme genes. B) A new RNAi strain containing the *ugt-27* plasmid has no effect on lifespan. Survival of *rrf-3* worms (20°C) on the control HT115(DE3) strain with the L4440 plasmid, (n=61), the original *ugt-27* strain (mutant) (n=98) and a new strain consisting of HT115(DE3) transformed with the *ugt-27* plasmid (n=50). Difference between mutant and control, 29.3% (P<.0001). C) The mutant *E*. *coli* strain extends lifespan without the plasmid. Survival of *rrf-3* (25°C) on the mutant strain from which the *ugt-27* has been lost. (n=68) compared with the control strain in which the L4440 empty vector has been lost (n=62). Increase in lifespan, 49.6% (P=<.0001).

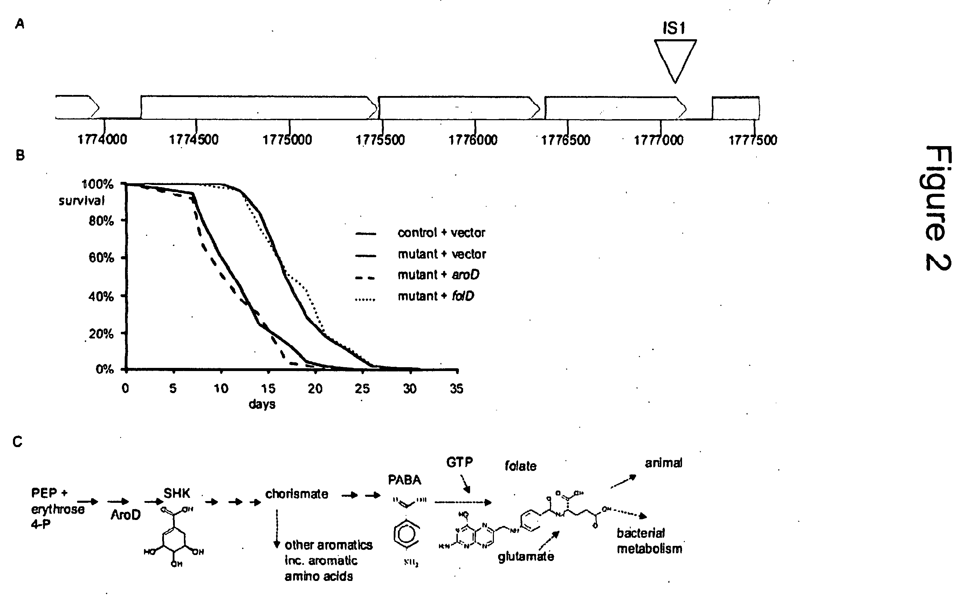

**Figure 2****.** Identification of the *E. coli* gene. A) Position of the IS1 transposon insertion at position 1777116 on the *E*. *coli* K12 W3110 chromosome29 (diagram based on EcoCyc.org30) Insertion at nucleotide 717 of the *aroD* open reading frame is thus designated *aroD717::IS*1. B) Schematic of shikimic acid pathway and folate synthesis. Solid arrows represent single enzymatic steps. Dashed arrows represent multiple steps. PEP, phosphoenolpyruvate; SHK, shikimic acid; PABA, paraaminobenzoic acid. C) The lifespan effect of the mutant bacteria is rescued by the plasmid containing *aroD* but not by another plasmid isolated in the screen (containing *folD*). *glp-4(bn1)* animals were raised on the *aroD* mutant until L4 (15°C) and then transferred to the *aroD* mutant + pMMB67EH vector (n=126), *aroD* mutant + pMMB67EH plasmid containing *aroD* region (n=85), *aroD* mutant + pMMB67EH plasmid containing *folD* region (n=121), wild type bacteria + vector (n=126) (25°C).

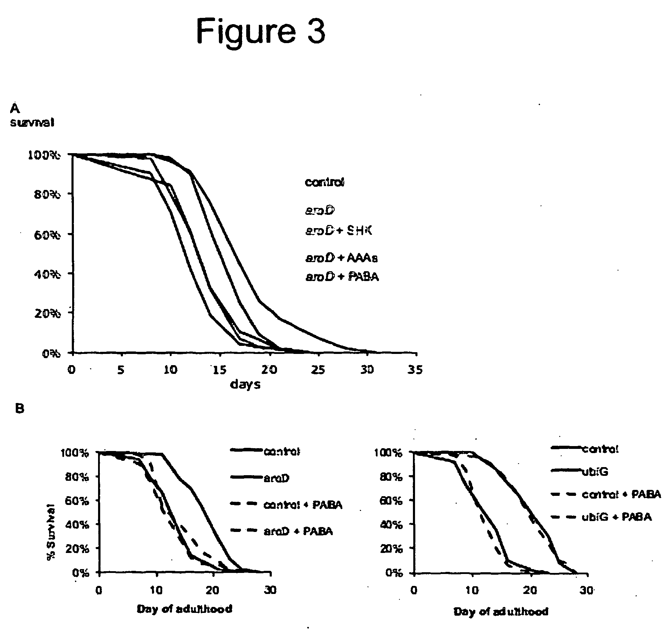

**Figure 3****.** Effect of media supplementation with shikimic acid and products of the shikimic acid pathway. A) *glp-4(bn1)* animals were raised on the *aroD* mutant until L4 (15°C) and then transferred to the *aroD* mutant (n=116), wild type control (n=116), *aroD* + shikimic acid (SHK, n= 86), *aroD* + PABA (n=76), or *aroD* + the aromatic amino acids (AAA, tyrosine, tryptophan and phenylalanine, n= 100) (25°C) *aroD* + PABA vs *aroD,* P < .0001; *aroD* + SHK vs *aro-,* P= <.0001. B) PABA supplementation reverses the lifespan extension on the mutant bacteria but has no effect on the control bacteria, *ubiG* bacteria or *ubiG-* + rescue plasmid. See Supplementary Table 1 for a full listing of all lifespan data in this study.

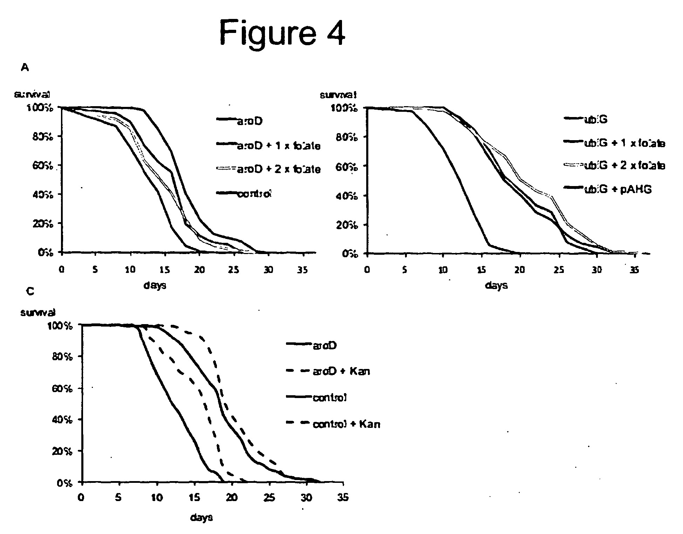

**Figure 4****.** Effect of media supplementation with folate on *C*. *elegans* lifespan on *aroD* and *ubiG* mutants. (A) *glp-4(bn1)* animals were raised on the *aroD* mutant until L4 (15°C) and then transferred to the *aroD* mutant (n=100), wild type control (n=126), *aroD* + 1 x folate (n=101), *aroD* + 2 x folate (n=149) (25°C). *aroD* vs *aroD* + 1 x folate, P < .0001; *aroD* + 1 x folate vs *aroD* + 2x folate, P= 0.13 (Log Rank), P=0.055 (Wilcoxon). (B) *glp-4* worms were raised on *E. coli* OP50 (15°C) and transferred at L4 to *ubiG* (n=145), *ubiG* + pAHG (*ubiG*+ rescue plasmid), GD1 + 1 x folate (n=145), GD1+ 2 x folate (n=146) (25°C). The control strains plus 1 x and 2 x folate were also tested (see Supp data). (C) *glp-4* worms were raised on OP50 (15°C) and transferred at L4 to *aroD,* control, *aroD* + kanamycin, control + kanamycin (25°C) (see Methods).
**Figure 5****.** (Supplementary Table 1). Summary of all lifespan experiments conducted in this study showing individual experiments, number of animals scored as dead, number censored and, where relevant, percent increase in lifespan between mutant and control, and P values from Log-Rank and Wilcoxon tests of the Kaplan-Meier survival model.
**Figure 6****.** *C*. *elegans* lifespan can be increased by inhibition of the bacterial folate pathway with sulfamethoxazole.
**Figure 7****.** Relationship between SMX concentration and *C*. *elegans* lifespan
**Figure 8****.** Folate partially reverses the increase in *C*. *elegans* lifespan caused by SMX
**Figure 9****.** The *aroD* mutation or SMX treatment decreases folate levels in bacteria and *C*. *elegans* maintained on the bacteria
**Figure 10****.** SMX has a minimal effect on *E. coli* growth

### Example 1: Decreased dietary folate from a microbial source increases C. elegans Lifespan

### Background and Summary

Diet has a strong effect on ageing and health but the key constituents are not defined and the mechanisms responsible are not understood. While a number of specific dietary constituents or manipulations have been postulated to increase human lifespan, none have yet been proven. Defining the precise relationship between diet and ageing and lifespan is challenging experimentally because of the complexity of diet, confounding environmental factors. Moreover, there is evidence that variations in the composition of gastrointestinal microbes can have large effects on the host animal. The metabolism of gastrointestinal microbes has a significant effect on nutrition.

As part of research towards increasing understanding of relationships between intestinal microbes, the animal and their diet, the inventors decided to study the *C*. *elegans*/*E. coli* model, which allows them to control the environment and genetics of both microbe and animal.

As set out below, from these studies the inventors have discovered that decreased bacterial folate synthesis slows animal ageing.

Folate supplementation of food reduces the occurrence of human birth defects but is controversial because of evidence of associated health risks. Folate is an essential enzymatic cofactor that animals must obtain from their diet or intestinal microbes. In the laboratory, the nematode *Caenorhabditis elegans* obtains folate from its diet of live *Escherichia coli.*

The present inventors have discovered an *E. coli* mutant that causes a substantial increase in *C*. *elegans* lifespan by decreasing folate supply to the animal. They found the causative mutation in the gene *aroD,* which encodes an enzyme functioning in the microbial shikimic acid pathway that generates aromatic compounds. The lifespan effect is eliminated by addition of one particular aromatic compound: para-aminobenzoic acid, the precursor of folate, thereby implicating decreased microbial folate synthesis as the cause of the increased longevity. Addition of folate, which can be absorbed by the animal but not the bacteria, also suppresses the lifespan extension, albeit incompletely, demonstrating that decreased dietary folate increases longevity. Folate is necessary for numerous synthetic reactions needed for growth.

Therefore decreased folate intake as a result of a poor diet or compromised intestinal microbial metabolism may trigger a nutrient-sensing mechanism that increases animal lifespan in conditions unfavourable for growth and reproduction.

### Research findings and conclusions

Defining the relationship between diet and ageing is challenging experimentally because of the complexity of diet and the heterogeneous microbial flora found in the gastrointestinal tract. Microbes assist animal nutrition by synthesising compounds essential to the host, such as folate, vitamins and amino acids. There is evidence that changes in the composition of microbes in the gastrointestinal tract influence nutrition-related syndromes such as obesity and are thus also likely to affect ageing. However, the mechanisms of these interactions are unknown. In lab studies, the diet of *C*. *elegans* is confined to a monoculture of the human gut bacteria *E. coli,* which must be alive to achieve maximal growth and reproduction, suggesting that microbial metabolic activity is critical for *C*. *elegans* nutrition. Limiting *E. coli* intake by various methods extends lifespan through dietary restriction (DR). However, none of these studies have identified which dietary components need to be restricted to increase lifespan. *E. coli* are also thought to reduce lifespan through pathogenesis of *C*. *elegans* because lifespan is increased on antibiotic or UV-treated *E. coli,* but these treatments may also work through effects on bacterial metabolism.

Expression of a specific *C*. *elegans* gene can be knocked down by feeding animals the HT115(DE3) *E*. *coli* strain containing a plasmid expressing double stranded RNA of the targeted gene (RNA-mediated interference, or RNAi). While using this method to test whether a class of genes influenced the lifespan of the long-lived *daf-2* insulin receptor mutant, the inventors noticed that one *E. coli* RNAi strain consistently caused a substantial (30-50%) increase in *C*. *elegans* lifespan (Figure 1A, Supplementary Table 1). The strain also extended the lifespan of wild-type *C*. *elegans* and mutants lacking the DAF-16/FOXO transcription factor that is required for *daf-2* mutant longevity (Supplementary Table 1, Supplementary Information). However, when the RNAi plasmid was isolated and retransformed into a fresh HT115(DE3) strain, the effect on lifespan disappeared (Figure 1B) even though the plasmid was still able to induce knockdown of the target gene (data not shown). Furthermore, when the plasmid was lost from the original *E*. *coli* strain by growth in the absence of selective antibiotic, the *E. coli* strain retained the ability to increase *C. elegans* lifespan (Figure 1C). Thus, the inventors had discovered a spontaneous mutant of the HT115(DE3) strain that extends *C. elegans* lifespan.

A previous study showed that *E. coli* mutants deficient in ubiquinone (coenzyme Q) synthesis cause *C*. *elegans* to live longer than wild type. This effect was originally attributed to lack of dietary Q, phenocopying the lifespan extension of the *clk-1* mutant that is defective in endogenous Q synthesis. However it was shown subsequently that lack of dietary Q is not responsible for the life-extending properties of the bacterial mutant. Instead, it has been suggested that lack-of bacterial respiration causes the animal to live longer. To see whether our spontaneous mutant had a respiratory defect the inventors tested whether it would utilise succinate as a carbon source, a process requiring respiratory metabolism. While the control bacteria grew on both succinate and the glucose control plates, the mutant was unable to grow on either carbon source. The minimal media used in these experiments lacked a number of amino acids (Supplementary Methods). Supplementation with these amino acids revealed that the addition of glycine supported weak growth of the mutant bacteria on both the glucose and succinate plates. This result suggests that the mutant is capable of respiration and extends lifespan by a mechanism distinct from the Q synthesis mutants. The inability of the mutant to grow on a defined media allowed screening for complementation of the mutated gene by DNA fragments from the wild type HT115(DE3) strain (see Supplementary Information). One plasmid from this screen containing the *aroD* and *ydiB* genes most effectively rescued mutant growth. PCR and DNA sequencing identified an IS1 transposon insertion in the *aroD* gene in the mutant bacteria that would lead to a truncated enzyme (Figure 2A). The *C*. *elegans* lifespan effect was rescued by transformation of the mutant bacteria with the plasmid containing *aroD* but not by one of the other isolated plasmids (Figure 2B). Thus, mutation of the *E. coli aroD* gene causes an increase in *C*. *elegans* lifespan.

*aroD* encodes the enzyme 3-dehydroquinate dehydratase, a core component of the shikimic acid pathway(Figure 2C). The end product of this pathway is chorismate, which acts as a precursor to all aromatic compounds in the bacterial cell, including the aromatic amino acids, the folate precursor para-amino benzoic acid (PABA) and quinones. Animals do not have this pathway and depend on their diet for many of its end products. Consistent with the involvement of this pathway in the extension of *C*. *elegans* lifespan, shikimic acid supplementation of the media caused *C*. *elegans* lifespan on *aroD* mutant bacteria to revert to normal (Figure 3A). The *aroD* mutant can grow on the rich peptone-based media used in this study, so the media must be able to provide either all the essential aromatic compounds needed for growth or the relevant precursors such as shikimic acid. It is therefore likely either one or more products of the shikimic acid pathway is present but in limiting supply, resulting in the lifespan effect. To find out whether one particular product of the pathway is important for the lifespan effect, the inventors added aromatic compounds known to support growth of *aro* mutants: PABA and the aromatic amino acids. The aromatic amino acids had a small negative effect on *C*. *elegans* longevity (Figure 3A), an effect due to tryptophan rather than phenylalanine or tyrosine (Supplementary Table 1). By contrast, PABA reversed the lifespan increase completely (Figure 3A, B). PABA supplementation had no effect on *C*. *elegans* on the control HT115(DE3) strain or on the extended lifespan of *C*. *elegans* maintained on the Q-deficient *ubiG* mutant bacteria (Figure 3B), ruling out a toxic effect of PABA. This result suggests that a decrease in folate synthesis in the *aroD* mutant bacteria is critical to the lifespan effect on *C*. *elegans*.

Folate is an essential coenzyme in all cells across all kingdoms so folate must be present in the *aroD* bacteria to enable bacterial growth. In addition, there must be sufficient folate produced by the bacteria to support growth of *C*. *elegans* because animals are incapable of making their own folate. Thus, a decrease in bacterial folate could increase *C*. *elegans* lifespan either by decreasing folate-dependent metabolism in *E*. *coli* or decreasing folate uptake in *C*. *elegans*. Exogenous folate cannot be taken up by *E. coli* but can be taken up by *C*. *elegans* and thus any effects of exogenous folate can be attributed to effects in the animal and not in the bacteria. Folate supplementation at the same molar concentration as PABA reduced the *C*. *elegans* lifespan extension substantially but not completely (Figure 4A, Supplementary Table 1). Because folate uptake from the media may be less efficient than uptake of bacteria-derived folate, the inventors tested whether doubling the folate concentration would suppress the lifespan extension further. Although the lifespan was slightly shorter (Figure 4A) the difference did not reach statistical significance. There were no toxic effects of folate at either concentration because these supplements had no effect on the lifespan of *C*. *elegans* maintained on either the control HT115(DE3) strain or the *ubiG* bacteria (Figure 4B, Supplementary Table 1). Thus, the increased longevity of *C*. *elegans* maintained on the *aroD* mutant is largely caused by decreased folate uptake. This result also demonstrates that the component of the lifespan increase suppressed by folate is not caused by inhibition of a negative effect of the bacteria, such as pathogenesis. To test this conclusion further the inventors examined the effect of kanamycin, which is thought to extend lifespan by suppressing bacterial pathogenesis Kanamycin treatment of wild-type *E. coli* increased *C*. *elegans* lifespan but not to the same degree as the *aroD* mutant and the presence of kanamycin did not interfere with the ability of the *aroD* mutant to increase *C*. *elegans* lifespan (Figure 4C). Thus the *aroD* mutant extends lifespan through a mechanism distinct from that of kanamycin. The incomplete effect of folate on the increased *C*. *elegans* lifespan on the *aroD* mutant suggests that a component of the lifespan extension is caused by repressing folate-dependent bacterial metabolism.

Using a simplified animal/microbe system the inventors have shown that a microbial mutation in the *aroD* gene can cause an increase in *C. elegans* lifespan. They have identified a specific dietary component, folate, that when restricted increases lifespan. As folate is essential for growth and reproduction, animals may correlate low folate levels with a poor diet and a decreased nutritional status of their intestinal microbes. Adaptation in response to decreased folate would increase survival until more nutrition can be found. Methionine restriction has been shown to extend lifespan in rodents and *Drosophila.* Folate is required for the conversion of homocysteine to methionine and thus decreased folate levels may result in methionine restriction. Interestingly, *C*. *elegans* lifespan can be increased by RNA interference of *sams-1*, the gene that encodes *C*. *elegans* S-adenosyl methionine synthase. This enzyme uses methionine to generate S-adenosyl methionine, a universal methyl donor used in several metabolic pathways. The inventors study may have uncovered a universal nutrient-sensing pathway in which the interaction between animals and their gastrointestinal microbes influences nutrition and ageing.

### Methods used

### C. elegans strains used

GA303 *rrf-3(pk 1426); daf-2(m577),* GR1307 *daf-16(mgDf50),* N2 (wild type), NL2099 *rrf*-*3*(*pk1426*), SS104 *glp-4(bn1).*

### E. coli strains

OP50 *ura,* HT115(DE3) is *W3110 rnc14::Tn10 Tn10(DE3 PlacUV5-T7 polymerase*), HT115(DE3) *aroD717.:*IS*1* this study, GD1 *ubiG,* GD1::pAHG (*ubiG*+).

### Culture conditions

Nematode growth media was prepared as described using 2.5 g/l soy peptone (Sigma P6713). Where indicated plates were supplemented with shikimic acid (40 µg/ml), I-phenylalanine, I-tryptophan and I-tyrosine (all at 40 µg/ml), 4-amino benzoic acid (PABA 40µg/ml = 0.29mM), sodium folate (0.29mM and 0.58mM folic acid dissolved in NaOH). For the kanamycin treatment of bacteria, 80 µl of 10 mM kanamycin was added after 24 hours of bacterial growth as described. All compounds were from Sigma-Aldrich.

### Lifespan analysis

For all lifespans and unless indicated differently in Supplementary Table 1, survival analyses were performed by the following method: Eggs were prepared from bleaching adults to remove all microbes, and then placed onto plates containing *aroD* mutant bacteria. Animals were raised at 15°C until adulthood due to the temperature sensitivity of mutant phenotypes. Gravid adulthoods were used to lay eggs onto fresh *aroD* mutant plates. At L3/L4 these animals were transferred to 25°C and larvae of equivalent stage were put onto at least 5 plates of 25 worms for each condition. Animals were transferred to fresh plates after 7 and 14 days and scored for survival every 2-3 days. Lifespan data was analysed by JMP statistical software (SAS). Where relevant, statistical significance was determined using the Log-Rank and Wilcoxon tests of fitting to the Kaplan-Meier survival model.

### Supplementary Information

### Characterisation of the life-extending effect of the mutant strain

All lifespan experiments conducted in this study are summarised in Supplementary Table 1. The mutant was discovered because it extended the lifespan of *rrf-3(pk1426); daf-2(m577)* mutants. The inventors then tested wild type *C*. *elegans* (N2), *rrf-3*(*pk1426*) mutants, *daf-16(mgDf50)* mutants and temperature-sensitive sterile mutants *glp*-*4*(*bn1*), shifted from 15°C to 25°C at L4. The mutant bacteria extended lifespan of all *C*. *elegans* strains. The effect at 25°C was stronger than at 20°C. To test whether the mutant bacteria exerted its effect during adulthood, the inventors shifted animals on mutant bacteria onto wild type bacteria, and *vice versa,* just before the beginning of adulthood as L4 larvae. Shifting from mutant bacteria to wild type bacteria caused C. *elegans* to have a wild type lifespan. Shifting in the other direction from wild type to mutant increased lifespan but took several days to have an effect, probably because residual wild type bacteria chemically complement the mutant bacteria with secreted PABA. These experiments imply that the effect of the bacteria on lifespan is exerted during adulthood. In all subsequent experiments, animals were raised on the mutant bacteria and then transferred to the experimental conditions at L4/young adult. Although the mutant occurred in a strain from the Ahringer RNAi library, the mutation occurred during culture in our laboratory. The inventors have tested the original *ugt-27* strain in the Ahringer library and it does not have the mutation.

### Gene identification

To identify the mutated gene the inventors took advantage of the inability of the mutant bacterial strain to grow on minimal media plates. The inventors partially digested the genomic DNA of the control strain using the 4-base cutter *Bfu*Cl that leaves *Bam*H1 compatible ends and ligated the fragments in a *Bam*HI-digested pMMB67EH, a low copy IncQ plasmid. They transformed the ligation mixture into the mutant strain and screened for large colonies on minimal media plates. Untransformed dead bacteria provided enough nutrients to support the growth of small colonies of transformed mutant bacteria, allowing estimation of the numbers screened. Colonies that were clearly larger than their neighbours were picked, grown up and the plasmid isolated by miniprep. The plasmids we rested by transformation into the mutant bacteria to test for enhanced growth on minimal media. From over 6000 colonies, six independent plasmids passed this second round of screening. Sequencing revealed that one containing the gene *folC,* one containing *folD,* three containing *aroK* and one containing *aroDlydiB.* This latter plasmid had the largest effect on mutant growth. The other plasmids appeared to have a positive effect on growth of both mutant and control strains. PCR and sequencing of the *aroD* region identified an IS1 transposon inserted in the *aroD* gene in the mutant strain but not in the control.

### Composition of minimal media

15 g/l agar, 2 g/l D-glucose, 2.17 g/l Na₂HPO₄, 1.35 g/l KH₂PO₄, 0.5 g/l (NH₄)₂SO₄, 0.01 g/l Ca(NO₃)₂, 0.005 g/l thiamine, 0.088g/l adenine, 0.044 g/l arginine, 0.11 g/l asparagine, 0.088 g/l cysteine, 0.11 g/l glutamatic acid, 0.088 g/l histidine, 0.066 g/l isoleucine, 0.133 g/l leucine, 0.066 g/l lysine, 0.088 g/l methionine, 0.066 g/l phenylalanine, 0.422 g/l serine, 0.221 g/l threonine, 0.088g/l tryptophan, 0.044 g/l tyrosine, 0.088 g/l. uracil, 0.166 g/l valine. 1% Trace element solution (5 g/l EDTA, 0.5 g/l FeCl₃, 0.05g/l ZnO, 0.01 g/l CuCl₂, 0.01 g/l CoCl₂.6H₂O, 0.01 g/l H₃BO₃)

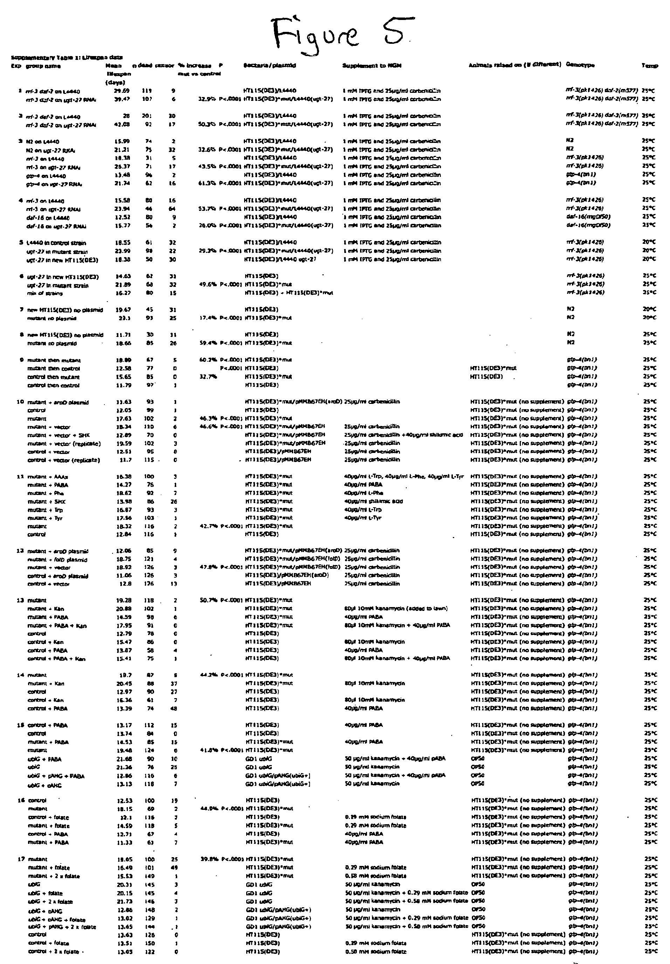

### Supplementary Table 1 (Figure 5)

See Figure 5 legend.

### Example 2: sulfamethoxazole treatment

Using the experimental procedures outlined herein the inventors examined whether sulfamethoxazole affects *C*. *elegans* lifespan. Sulfamethoxazole (SMX) is a sulphonamide that competes with the folate-precursor PABA; it is a competitive inhibitor of dihydropteroate synthase (DHPS).

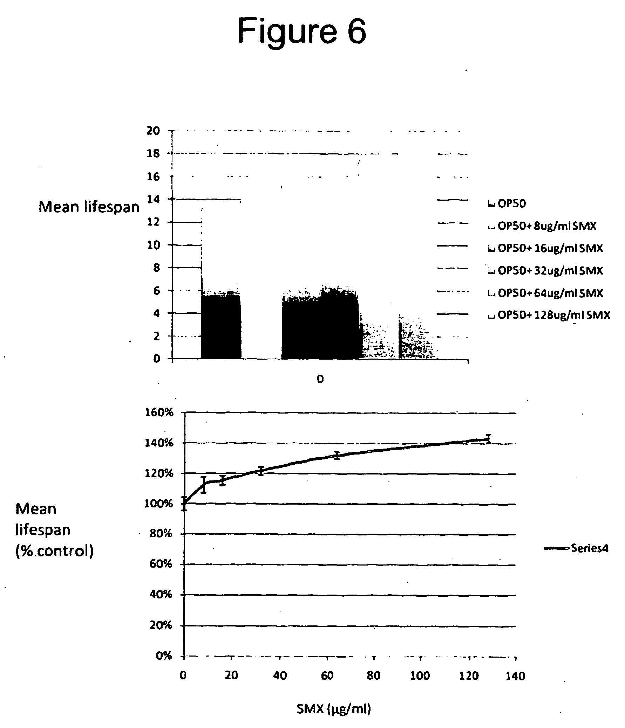

The data from these experiments are presented in Figure 6. Here it can be seen that *C*. *elegans* lifespan can be increased by inhibition of the bacterial folate pathway with sulfamethoxazole.

Accordingly, sulfamethoxazole is an inhibitor of microbial folate biosynthesis and can be used to slow ageing.

### Example 3: Further experimental data supporting the invention.

Using the experimental procedures outlined herein the inventors have generated additional data in support of the present invention.

### 1. Further dose-response data for SMX

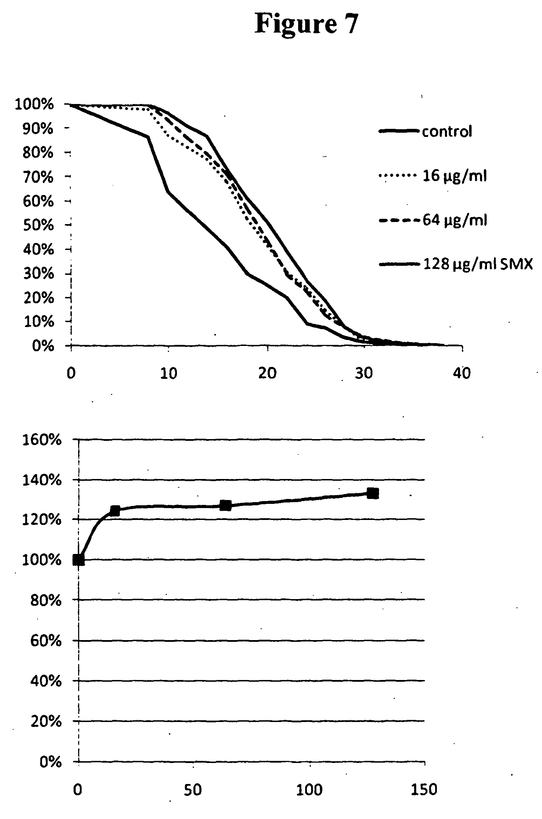

The inventors have continued to refine the dose response relationship between SMX treatment of the bacteria and increases in *C*. *elegans* lifespan. Further lifespan analyses suggest that the lifespan effect occurs with a two-phase response (See Figure 7). 16 µg/ml SMX causes a significant and large increase in *C*. *elegans* lifespan of around 24% (n=207 (control), n=240 (16 µg/ml SMX), p = <0.0001). However, four times that concentration (64 µg/ml) has only a small further increase in lifespan to 27% (n=256), which is not significantly different to the effect caused by 16 µg/ml. Doubling the concentration to 128 µg/ml leads to a second phase in which the lifespan is increased to 33% more than the control, and is significantly different to the effect caused by 16 µg/ml (n=274, p = <0.05). These results imply that a large effect on lifespan can be achieved at low doses (around 16 µg/ml) and perhaps sets a starting point for application to mammalian species.

### 2. Folate rescues SMX effect

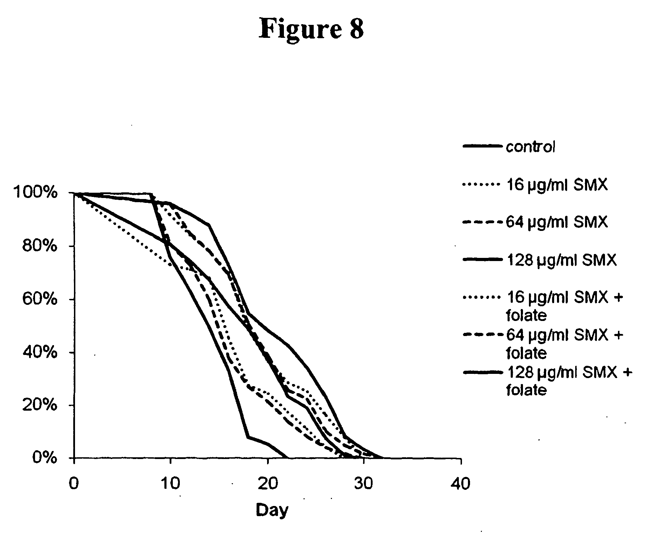

Adding folic acid to the SMX plates reverses the effect of SMX (Figure 8). This result and the similarity with the effect of folic acid on the *aroD* mutation (Figure 4), provides further supporting data that SMX is working by inhibiting folate synthesis rather than through a secondary effect.

### 3. Folate levels changes in bacteria caused aroD and SMX. Results in changes in worm folates

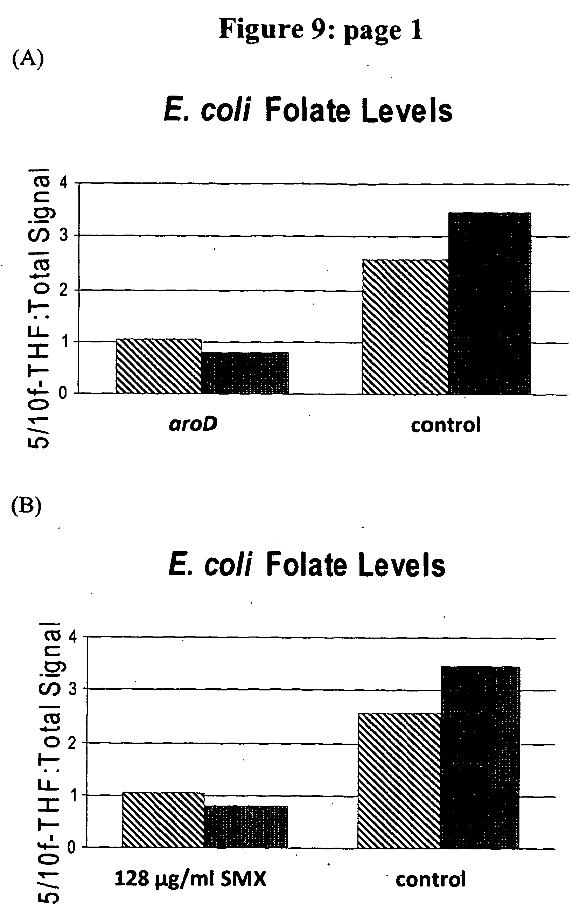

Both the *aroD* mutation and treatment with SMX inhibit folate synthesis in bacteria. Using HPLC/MS analysis based on published methods the inventors have found that formyl tetrahydrofolate triglutamate is the most abundant folate species in *E*. *coli* grown in the experimental conditions. This folate species is approximately three fold less abundant in the *aroD* mutant compared to the control strain (in duplicate experiments, Figure 9A) and at least 10 times less abundant in the SMX-treated OP50 bacteria compared to the untreated control (in duplicate experiments, Figure 9B).

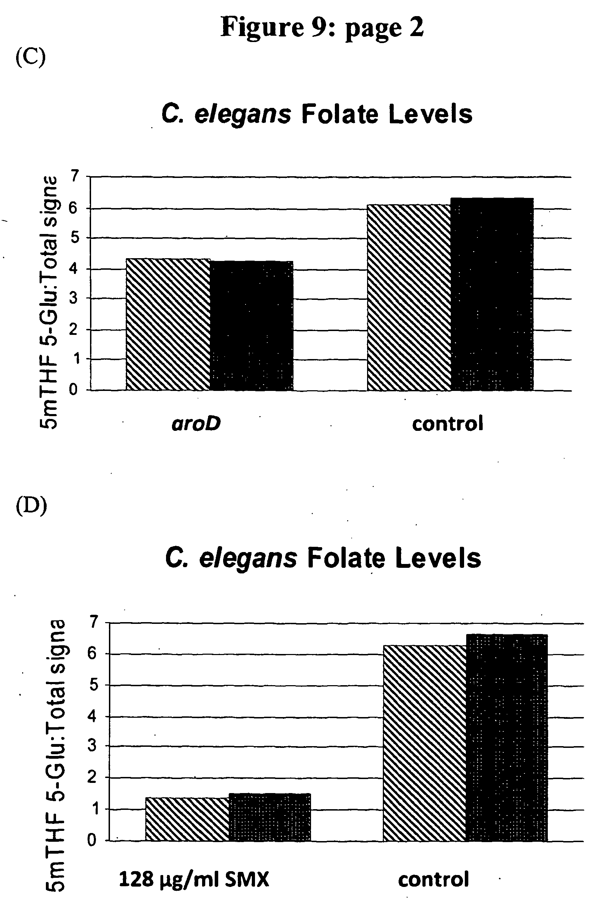

Similar techniques were used to quantify levels of folates in worms, although proteinase K digestion of the worms was used to get a good extraction of folates. The most abundant folate species in *C*. *elegans* using this method of detection was 5-methyl tetrahydrofolate pentaglutamate. Worms raised on *aroD* bacteria had 25 to 30% less of this folate species than worms on the control bacteria (in duplicate experiments, Figure 9C). Worms raised OP50 bacteria treated with SMX had approximately 75% less folate than worms raised on the control bacteria (in duplicate experiments, Figure 9D).

### 4. SMX has a minimal effect on bacterial growth

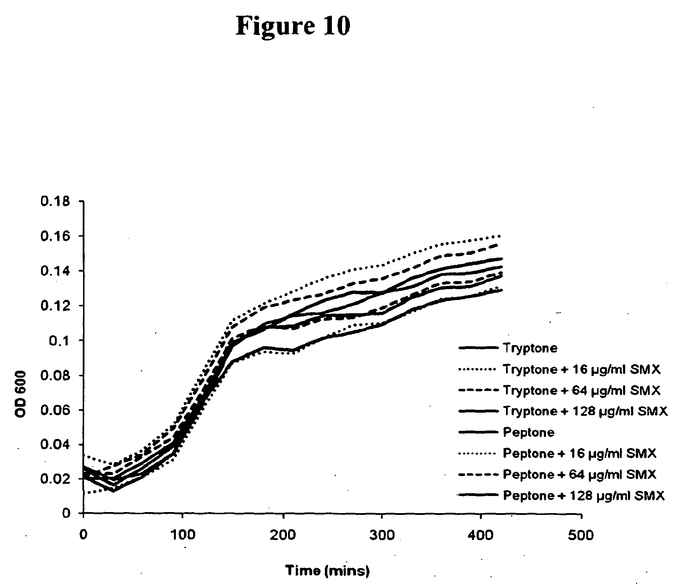

The inventors investigated the effect of SMX on *E*. *coli* growth. Under the conditions that caused an increase in *C*. *elegans* lifespan, they scraped the *E*. *coli* from the agar plates used to culture worms after two days of growth and used absorbance to estimate relative cell numbers. 128 µg/ml SMX caused a mean effect of reducing *E*. *coli* numbers by approx 11% (+/- 5%, standard error, n=4). There was no effect on growth in liquid culture with the same nutrient conditions as used for lifespan analysis (Figure 10). Although SMX is used as an antibiotic in the clinic, it is only used in combination with the dihydrofolate reductase inhibitor trimethoprim as co-trimoxazole.

The effect of SMX on *C*. *elegans* lifespan seems to not to work through inhibition of bacterial proliferation as is thought be the case for kanamycin or carbenicillin (Garigan, D., et al (2002) Genetics 161, 1101-1112), but rather as a consequence of partial inhibition of bacterial folate inhibition. The subtlety of this effect implies that long-term treatment of SMX or similar drug can be achieved without having a large effect on the ecology of intestinal microbes, thereby avoiding some of the issues of antibiotic treatment.

## Claims

1. An *in vitro* method of screening for an agent for use as a medicament for slowing ageing comprising determining whether a test agent inhibits microbial folate biosynthesis.

2. The method of claim 1 comprising the steps of (i) contacting a population of micro-organisms with a quantity of the test agent; and (ii) determining the effect of the test agent on microbial folate biosynthesis.

3. The method of claim 2 wherein the micro-organism is a member of the animal gastrointestinal tract flora.

4. A non-therapeutic use of an inhibitor to reduce the activity of an enzyme in the folate biosynthesis pathway for slowing ageing, wherein:
(i) the enzyme is 3-dehydroquinate dehydratase; or
(ii) the enzyme is dihydropteroate synthase (DHPS) and the inhibitor is *p-*aminosalicylic acid.

5. A non-therapeutic method of slowing ageing, the method comprising administering an inhibitor of an enzyme in the folate biosynthesis pathway, wherein:
(i) the enzyme is 3-dehydroquinate dehydratase; or
(ii) the enzyme is dihydropteroate synthase (DHPS) and the inhibitor is *p-*aminosalicylic acid.

6. A composition comprising an inhibitor of microbial folate biosynthesis and one or more further agents capable of slowing ageing, wherein said one or more further agents capable of slowing ageing is resveratrol; acetyl-L-carnitine; alpha-lipoic acid; MK-677; and/or rapamycin.

7. The composition according to claim 6 wherein the inhibitor reduces the activity of an enzyme in the folate biosynthesis pathway.

8. The composition according to claim 7 wherein the enzyme is 3-dehydroquinate dehydratase.

9. The composition according to claim 7 wherein the enzyme is dihydropteroate synthase (DHPS), wherein the inhibitor is preferably a sulphonamide, such as sulfamethoxazole; or wherein the inhibitor is p-aminosalicylic acid.

10. A food supplement, additive, functional food, or nutraceutical comprising the composition according to any of claims 6-9.

## Patentansprüche

1. *In*-*vitro*-Verfahren zum Screening nach einem Mittel zur Verwendung als Medikament zwecks Verlangsamung von Alterung, umfassend Feststellen, ob ein Testmittel mikrobielle Folatbiosynthese hemmt.

2. Verfahren nach Anspruch 1, umfassend die Schritte (i) In-Kontakt-Bringen einer Population von Mikroorganismen mit einer Menge des Testmittels; und (ii) Feststellen der Wirkung des Testmittels auf mikrobielle Folatbiosynthese.

3. Verfahren nach Anspruch 2, wobei der Mikroorganismus ein Mitglied der Flora des Gastrointestinaltrakts eines Tieres ist.

4. Nicht therapeutische Verwendung eines Hemmers zum Verringern der Aktivität eines Enzyms im Folatbiosyntheseweg zwecks Verlangsamung von Alterung, wobei:
(i) das Enzym 3-Dehydrochinat-Dehydratase ist; oder
(ii) das Enzym Dihydropteroat-Synthase (DHPS) ist und der Hemmer *p-*Aminosalicylsäure ist.

5. Nicht therapeutisches Verfahren zur Verlangsamung von Alterung, wobei das Verfahren Verabreichen eines Hemmers eines Enzyms im Folatbiosyntheseweg umfasst, wobei:
(i) das Enzym 3-Dehydrochinat-Dehydratase ist; oder
(ii) das Enzym Dihydropteroat-Synthase (DHPS) ist und der Hemmer *p-*Aminosalicylsäure ist.

6. Zusammensetzung, umfassend einen Hemmer mikrobieller Folatbiosynthese und ein oder mehrere weitere Mittel mit der Fähigkeit zur Verlangsamung von Alterung, wobei das eine oder die mehreren weiteren Mittel mit der Fähigkeit zur Verlangsamung von Alterung Resveratrol; Acetyl-L-Carnitin; Alpha-Liponsäure; MK-677; und/oder Rapamycin ist bzw. sind.

7. Zusammensetzung nach Anspruch 6, wobei der Hemmer die Aktivität eines Enzyms im Folatbiosyntheseweg verringert.

8. Zusammensetzung nach Anspruch 7, wobei das Enzym 3-Dehydrochinat-Dehydratase ist.

9. Zusammensetzung nach Anspruch 7, wobei das Enzym Dihydropteroat-Synthase (DHPS) ist, wobei der Hemmer vorzugsweise ein Sulfonamid, wie Sulfamethoxazol, ist; oder wobei der Hemmer *p*-Aminosalicylsäure ist.

10. Nahrungsergänzungsmittel, Zusatzmittel, funktionelles Lebensmittel oder Nutrazeutikum, das die Zusammensetzung nach einem der Ansprüche 6-9 umfasst.

## Revendications

1. Procédé *in vitro* de criblage d'un agent pour l'utilisation en tant que médicament pour ralentir le vieillissement, comprenant la détermination du point de savoir si un agent d'essai inhibe la biosynthèse microbienne des folates.

2. Procédé selon la revendication 1, comprenant les étapes consistant (i) à mettre en contact une population de micro-organismes avec une quantité de l'agent d'essai; et (ii) à déterminer l'effet de l'agent d'essai sur la biosynthèse microbienne des folates.

3. Procédé selon la revendication 2, dans lequel le micro-organisme est un élément de la flore du tube digestif animal.

4. Utilisation non thérapeutique d'un inhibiteur pour réduire l'activité d'une enzyme dans le chemin de la biosynthèse des folates pour ralentir le vieillissement, dans laquelle:
(i) l'enzyme est la 3-déshydroquinate déshydratase; ou
(ii) l'enzyme est la dihydroptéroate synthase (DHPS) et l'inhibiteur est l'acide *p*-aminosalicylique.

5. Procédé non thérapeutique de ralentissement du vieillissement, le procédé comprenant l'administration d'un inhibiteur d'une enzyme dans le chemin de la biosynthèse des folates, dans lequel:
(i) l'enzyme est la 3-déshydroquinate déshydratase; ou
(ii) l'enzyme est la dihydroptéroate synthase (DHPS) et l'inhibiteur est l'acide *p*-aminosalicylique.

6. Composition comprenant un inhibiteur de la biosynthèse microbienne des folates et un ou plusieurs agents supplémentaires capables de ralentir le vieillissement, dans laquelle lesdits un ou plusieurs agents supplémentaires capables de ralentir le vieillissement sont le resvératrol; l'acétyl-L-carnitine; l'acide alpha-lipoïque; MK-677; et/ou la rapamycine.

7. Composition selon la revendication 6, dans laquelle l'inhibiteur réduit l'activité d'une enzyme dans le chemin de la biosynthèse des folates.

8. Composition selon la revendication 7, dans laquelle l'enzyme est la 3-déshydroquinate déshydratase.

9. Composition selon la revendication 7, dans laquelle l'enzyme est la dihydroptéroate synthase (DHPS), dans laquelle l'inhibiteur est de préférence un sulfonamide, comme le sulfaméthoxazole; ou dans laquelle l'inhibiteur est l'acide p-aminosalicylique.

10. Complément alimentaire, additif, aliment fonctionnel ou nutraceutique comprenant la composition selon l'une quelconque des revendications 6 à 9.
